# EUROPÄISCHE PATENTSCHRIFT

(11) **EP 0 433 420 B1**
(45) Veröffentlichungstag und Bekanntmachung des Hinweises auf die Patenterteilung: **09.03.1994**
(21) Anmeldenummer: 90909722.2
(22) Anmeldetag: 22.06.1990
(51) Int. Cl.: C07C 231/02

(54) **VERFAHREN ZUR HERSTELLUNG SYMMETRISCHER DIFETTSÄUREDIAMIDE**
PROCESS FOR MAKING SYMMETRICAL DI-FATTY ACID DIAMIDES
PROCEDE DE PRODUCTION DE DIAMIDES D'ACIDES DIGRAS SYMETRIQUES

(30) Priorität: 29.06.1989 DE 3921342
(43) Veröffentlichungstag der Anmeldung: 26.06.1991
(73) Patentinhaber: Dr. Frische GmbH, D-63755 Alzenau (DE)
(72) Erfinder: FRISCHE, Rainer, D-6000 Frankfurt am Main (DE); VOLKHEIMER, Jürgen, D-6200 Wiesbaden (DE); WOLLMANN, Klaus, D-6251 Eschhofen (DE); SCHOMANN, Herrmann, D-6070 Langen (DE); SCHNEIDER, Judith, D-6000 Frankfurt am Main (DE); ACH, Alexander, D-6000 Frankfurt am Main (DE); GROSS-LANNERT, Renate, D-6057 Dietzenbach (DE); BEST, Bernd, D-6082 Mörfelden (DE)
(74) Vertreter: Reichel, Wolfgang, Dipl.-Ing.
(86) Internationale Anmeldenummer: EP9000996
(87) Internationale Veröffentlichungsnummer: WO9100263

(56) Entgegenhaltungen:
- DE-A- 1 905 550
- GB-A- 558 854
- GB-A- 632 242
- CHEMICAL ABSTRACTS, Band 80, Nr. 15, 15. April 1974, Columbus, Ohio, US; Zusammenfassung Nr. 82134k, Seite 330 & JP-A-7 343 089

## Beschreibung

Die Erfindung bezieht sich auf ein Verfahren zur Gewinnung von symmetrisch zusammengesetzten Difettsäurediamiden.

Difettsäurediamide sind technisch vielseitig verwendbare Verbindungen, die unter anderem als Hilfsstoffe in der Kunststoffverarbeitung, als Schmierstoffadditive oder als Flußmittel für Lote eingesetzt werden. Solche Difettsäurediamide werden im wesentlichen aus aktivierten Fettsäuregemischen marktgängiger Öle durch Umsatz mit Diaminen hergestellt. Die aus derartigen Umsetzungen erhaltenen Diamide stellen chemisch gesehen keine einheitlichen Verbindungen dar. Vielmehr handelt es sich hierbei um Gemische von Difettsäurediamiden, wie sie entsprechend den verschiedenen Kombinationsmöglichkeiten in Abhängigkeit von der Fettsäureverteilung in den eingesetzten Rohstoffen entstehen. Ausgehend von natürlichen Fetten und Ölen, die drei verschiedene Fettsäuren enthalten, entstehen demzufolge nach den Regeln der Kombinatorik Gemische aus sechs verschiedenen Difettsäurediamiden, die teils symmetrisch, d.h. bestehend aus zwei gleichartigen Fettsäureresten, teils asymmetrisch aufgebaut sind. Derartig heterogen zusammengesetzte Difettsäurediamidgemische können daher nur in solchen Fällen eingesetzt werden, bei denen diese Heterogenität nicht stört oder von Vorteil ist. Dagegen wäre es beispielsweise vorteilhaft, wenn als Gleitvermittler für Kunststoffe, wie sie u.a. bei der Ummantelung von Kabeln verwendet werden, die reinen Diölsäurediamide eingesetzt werden könnten.

Die Trennung von Fettsäuregemischen ist allgemein mit erheblichem Aufwand verbunden. Die Gründe hierfür liegen u.a. in der strukturellen Ähnlichkeit der zu trennenden Fettsäuremoleküle oder in der Reaktivität der häufig vorhandenen Doppelbindungen. Die Trennung gestaltet sich um so schwieriger, je reiner das erhaltene Produkt sein soll. Besondere Probleme treten bei der Auftrennung von Fettsäuren gleicher Kettenlänge auf, die sich nur in der Anzahl der Doppelbindungen voneinander unterscheiden. Einen solchen Fall stellen z. B. die technisch sehr interessanten C₁₈-Carbonsäuren Stearinsäure, Ölsäure, Linolsäure und Linolensäure dar. So ist die Reindarstellung der Ölsäure aus Naturfetten sehr mühsam, da die restlose Abtrennung von anderen gesättigten und ungesättigten Fettsäuren nur schwer zu erreichen ist. Entsprechend ist das klassische Verfahren zur Darstellung reiner Ölsäure durch Tieftemperatur-Kristallisation von Olivenölfettsäuren oder deren Methylestern aufwendig und verlustreich. Gerade am Beispiel der Ölsäure läßt sich jedoch zeigen, daß ein höherer Gehalt dieses Bestandteils, z.B. in kosmetischen Erzeugnissen wie Sonnenmilch, die Qualität dieser Produkte erheblich verbessern könnte.

Aufgabe der vorliegenden Erfindung war es daher, ein Verfahren zur Verfügung zu stellen, mit dessen Hilfe es möglich ist, die symmetrisch zusammengesetzten Difettsäurediamide in so reiner Form zu erhalten, so daß sie sowohl als Grundstoff für höherwertige Produkte, beispielsweise zur Herstellung von Kunststoffen, wie sie in der gleichzeitig eingereichten Patentanmeldung DE 40 19 087 beschrieben werden, als auch als Ausgangsstoff für die Reindarstellung von auf andere Weise nur schwer erhältlichen Fettsäuren geeignet sind.

Diese Aufgabe wurde erfindungsgemäß durch ein Verfahren nach Anspruch 1 gelöst.

Überraschend wurde gefunden, daß sich Löslichkeiten und Kristallisierungsgeschwindigkeiten von Diamiden gesättigter, ungesättigter und hydroxylierter Fettsäuren in organischen Lösungsmitteln viel stärker voneinander unterscheiden, als dies bei den entsprechenden freien Säuren der Fall ist. Die Löslichkeit hängt hierbei in entscheidendem Maße von den funktionellen Gruppen innerhalb der Fettsäuremoleküle ab und folgt einigen grundlegenden Regeln.

Es wurde gefunden, daß eine OH-Gruppe innerhalb eines Fettsäuremoleküls generell eine sehr schlechte Löslichkeit des entsprechenden Diamids im Vergleich mit Diamiden gesättigter und ungesättigter Fettsäuren bewirkt. Beispielsweise sind die Difettsäurediamide, die 12-Hydroxystearinsäure enthalten, wie sie in hydriertem Ricinusöl vorkommt, selbst in siedendem Methanol praktisch nicht mehr löslich. Die Zahl der Hydroxygruppen beeinflußt die Löslichkeiten außerdem so stark, daß sich Dihydroxyverbindungen wie Bis-12-hydroxystearinsäurediamide bequem von denjenigen Diamidderivaten abtrennen lassen, die nur einen hydroxylgruppenhaltigen Fettsäurerest besitzen. Eine zusätzliche Doppelbindung, wie sie in der Ricinolsäure vorkommt, verbessert die Löslichkeit deutlich. Weiter wurde beobachtet, daß in Abhängigkeit von der Anzahl der Doppelbindungen im Fettsäuremolekül die Löslichkeit der entsprechenden Diamide im Vergleich zu den Diamiden der entsprechenden gesättigten Fettsäuren ansteigt. Dies ermöglicht eine problemlose Trennung der ungesättigten Fettsäuren sowohl von gesättigten wie von hydroxylgruppentragenden Fettsäuren. Hierbei sind die Unterschiede so bedeutsam, daß sich, obwohl die Löslichkeit der Fettsäuren i.a. mit abnehmender Kettenlänge zunimmt, sogar relativ kurzkettige gesättigte Fettsäuren wie die Laurinsäure, die stark angereichert in Palmkern- und Cocosfett vorkommt, in Form ihrer Diamide noch gut von C₁₈-ungesättigten Fettsäuren abtrennen lassen. Ferner wurde gefunden, daß symmetrisch aufgebaute Difettsäurediamide verbunden mit einer höheren Kristallisationsgeschwindigkeit eine schlechtere Löslichkeit aufweisen als asymmetrisch aufgebaute Difettsäurediamide, sofern es sich bei den jeweiligen Bestandteilen der Diamide um Fettsäuren mit vergleichbaren Löslichkeitseigenschaften handelt. Die Löslichkeit von Diamiden, die sich aus gesättigten und ungesättigten Fettsäuren zusammensetzen, liegt zwischen derjenigen von Diamiden, die nur aus gesättigten bzw. nur aus ungesättigten Fettsäuren aufgebaut sind.

Eine weitere überraschende Erkenntnis war, daß sich aus Ölen und Fetten, die eine starke Dominanz einer Fettsäure im Fettsäuremuster aufweisen, überproportional hohe Mengen einheitlich zusammengesetzter Difettsäurediamide bilden. Hierbei spielt die Art der Fettsäure keine Rolle. Geht man beispielsweise von Fettsäurekomponenten aus, in denen die Ölsäure zu 50% vertreten ist, so beträgt der Anteil des Diölsäurediamids unter den gebildeten Difettsäurediamiden maximal 25%. Bei einem Ölsäuregehalt von ungefähr 70%, dem für marktgängige Produkte üblichen Anreicherungsgrad, bildet sich das Diölsäurediamid maximal zu 49%. Ein Difettsäurediamidgemisch enthält etwa 80% einheitlich zusammengesetztes Diölsäurediamid, wenn von Triglyceriden ausgegangen wird, in denen Ölsäure im Fettsäuremuster zu 90% vertreten ist.

Diese Erkenntnisse lassen sich nun nach dem erfindungsgemäßen Verfahren einerseits besonders vorteilhaft zur Herstellung symmetrischer Difettsäurediamide aus Fettsäuregemischen nutzen, in denen eine Fettsäure eine starke Dominanz aufweist, und ermöglichen andererseits auf diesem Wege auch gleichzeitig die Aufkonzentrierung und die Reindarstellung seltener außergewöhnlicher Fettsäuren. Von besonderem Vorteil ist es, daß die Umsetzung von Gemischen von Fettsäureestern oder Fettsäuren mit Diaminen, wie in der gleichzeitig eingereichten Patentanmeldung DE 40 19 089 beschreiben, nicht nur mit vorgereinigten Fettsäureprodukten, sondern auch mit rohen Fetten und Ölen möglich ist, so daß auch Öle aus neuartigen Züchtungen wie Sonnenblumen mit hohem Ölsäuregehalt, insbesondere der Art "High Oleic", oder wie der Euphorbia lathyris, in der die Ölsäure mit einem Gehalt von etwa 85 % im Fettsäuremuster vorliegt, einer Weiterverarbeitung zugänglich sind. Im letzteren Fall enthält das sich bildende Difettsäurediamidgemisch ungefähr 70% Diölsäurediamid.

Für das erfindungsgemäße Verfahren können grundsätzlich beliebige Mischungen gesättigter, ungesättigter und hydroxylierter Fettsäuren und Fettsäureester eingesetzt werden. Besonders vorteilhaft werden Fette und Öle pflanzlichen oder tierischen Ursprungs als Ausgangsmaterialien eingesetzt, wobei das Verfahren insbesondere zur Umsetzung roher Fette und Öle geeignet ist, wie sie nach den üblichen Methoden, z.B. durch Kalt- oder Heißpressen mit Schnecken- oder Spindelpressen oder durch Pressextraktion gewonnen werden. Die in solchen rohen Ausgangsmaterialien unter Umständen vorhandenen festen Inhaltsstoffe, z.B. Holz oder Pflanzenreste, werden vor der Umsetzung zweckmäßig abgetrennt. Bevorzugt werden solche Fette und Öle eingesetzt, die einen besonders hohen Gehalt an funktionellen oder an außergewöhnlichen Fettsäuren aufweisen. Der Anteil an den gewöhnlichen funktionellen Fettsäuren sollte zweckmäßig wenigstens 50 %, der Anteil an außergewöhnlichen Fettsäuren wenigstens 10 % betragen, jeweils bezogen auf die Gesamtzahl der Fettsäuremoleküle. Besonders bevorzugt werden das Öl der Euphorbia lathyris, linol- und ölsäurereiches Sonnenblumenöl, insbesondere der Art "High Oleic", Ricinusöl oder hydriertes Ricinusöl, Leinöl und Rapsöle, insbesondere erucasäurereiches Rapsöl, das Öl der Purgiernuß, Olivenöl oder Seetieröle wie Fisch- oder Waltranöl verwendet. Die Menge eingesetzten Ausgangsmaterials ist beliebig, so daß das Verfahren grundsätzlich sowohl im Labormaßstab als auch im industriellen Maßstab durchführbar ist.

Die jeweiligen Ausgangsmaterialien können direkt mit den Diaminen umgesetzt werden. Als Diamine können z.B. primäre und sekundäre aliphatische, cycloaliphatische, aliphatisch-aromatische oder aromatische Diamine, vorzugsweise mit 2 bis 44 Kohlenstoffatomen, eingesetzt werden. Hierunter fallen beispielsweise auch dimere Fettsäuren aus natürlichen Fetten und Ölen. Zwischen den beiden Aminofunktionen der Diamine können sich in der Kohlenwasserstoffkette oder am cycloaliphatischen oder aromatischen Rest außerdem zusätzliche strukturelle Elemente bzw. weitere funktionelle Gruppen, z.B. Ethergruppen, Aminogruppen, Diamidgruppierungen, Ketogruppen oder Sulfongruppen befinden. Bevorzugte Diamine sind 1,2-Diaminoethan, 1,3-Diaminopropan, 1,6-Diaminohexan, 1,8-Diaminooctan, Piperazin, 4,7,10-Trioxatridecan-1,13-diamin, 3,3'-Diaminodiphenylsulfon, 3,3'-Dimethyl-4,4'-diaminodicyclohexylmethan und handelsübliche Etherdiamine der Formel 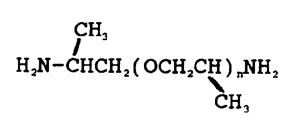
wobei n eine ganze Zahl von 1 bis 2000 ist. Besonders bevorzugte Verbindungen sind 1,2-Diaminoethan und 1,6-Diaminohexan.

Die Diamine werden, bezogen auf die Anzahl der Aminofunktionen und der Fettsäurereste, bevorzugt in stöchiometrischen Mengen eingesetzt, das Mischungsverhältnis ist jedoch nicht überaus kritisch, da sich auch bei einem zweifachen Überschuß an Aminofunktionen überraschenderweise die Diamide noch bevorzugt vor den Monoamiden bilden.

Gegebenenfalls kann die Umsetzung auch in einem geeigneten Lösungsmittel erfolgen, um eine homogene Reaktionsführung zu gewährleisten. In der Regel werden unpolare Lösungsmittel, insbesondere Toluol, Xylol oder Petrolether verwendet werden.

Die Umsetzung kann in einem Temperaturbereich zwischen 20 und 300°C erfolgen, bevorzugt wird jedoch ein Bereich zwischen 50 und 200°C, da in diesem Temperaturbereich die Reaktionszeiten mit 1 bis 6 Stunden für die praktische Durchführung des Verfahrens noch nicht zu lang sind.

Die Reaktion wird vorsorglich in geschlossenen Systemen, z.B. in einem Autoklaven, durchgeführt. Die Reaktion kann ohne besonderen Aufwand erfolgen, wird jedoch bevorzugt unter Inertgasatmosphäre, z.B. von Argon oder Stickstoff durchgeführt, da hierdurch mehr Sicherheit gegen unerwünschte Nebenreaktionen wie die Oxidation der Ausgangsmaterialien erreichbar ist.

Falls erforderlich, können dem Reaktionsgemisch auch Katalysatoren, z.B. Ammoniumchlorid oder p-Toluolsulfonsäure zugefügt werden. Auch biologische Katalysatoren wie Esterasen können bei den für diese Enzyme geeigneten Temperaturen eingesetzt werden. Ebenso können weitere übliche Hilfs- und Zusatzstoffe wie Polymerisationshemmer und Antioxidantien, z.B. Ascorbinsäure oder Glukose zugesetzt werden.

Nach Beendigung der Reaktion werden die Reaktionsprodukte durch einfache oder fraktionierte Kristallisation abgetrennt und, falls erforderlich, anschließend aus geeigneten Lösungsmitteln umkristallisiert. Als Lösungsmittel eignen sich sowohl polare wie unpolare Verbindungen. Bevorzugt wird aus Methanol oder Ethanol umkristallisiert. Unter Umständen genügt auch bereits ein einfacher Waschvorgang, beispielsweise mit Toluol oder Methanol, um reine Reaktionsprodukte zu erhalten. Eine besonders bevorzugte Reinigungsmethode stellt die Lösungsmittelheißdampf-Extraktion dar, bei der die Disäurediamide ebenfalls in kristalliner Form erhalten werden.

Die nach dem erfindungsgemäßen Verfahren hergestellten Difettsäurediamide werden mit einem so hohen Reinheitsgrad erhalten, daß sie problemlos weiterverarbeitet werden können. So lassen sich die Difettsäurediamide entweder direkt als Zusatzstoffe, z.B. als Schmierstoffadditive einsetzen oder aber in weitere interessante Folgeprodukte umwandeln. So können Difettsäurediamide, deren Fettsäusrereste funktionelle Gruppen wie Kohlenstoff-Kohlenstoff-Doppelbindungen oder OH-Gruppen tragen, beispielsweise die aus dem rohen Öl der Euphorbia-lathyris-Samen oder aus rohem Ricinusöl in hochkonzentrierter Form erhältlichen Diölsäurediamide bzw. Diricinolsäurediamide, wie in der gleichzeitig eingereichten Anmeldung DE 40 19 087 beschrieben, mit geeigneten bifunktionellen Verbindungen, z.B. mit Diisocyanaten, umgesetzt werden und stellen somit neuartige Grundstoffe zur Erzeugung von Präpolymeren, Kunststoffen und Kunststoffadditiven, z.B. für Klebstoffe, Dichtungsmassen, Kunststoffschaumstoffe, Schmierstoffe sowie eine Vielzahl weiterer technischer Hilfsstoffe dar. Aus den Difettsäurediamiden lassen sich schließlich nach den üblichen Verfahren der Verseifung auch die freien Fettsäuren herstellen, die ihrerseits wiederum derivatisiert werden können, oder aber, falls es sich um Fettsäuren mit weiteren funktionellen Gruppen handelt, selbst als Grund- oder Zusatzstoffe für Kunststoffe dienen können.

Erfindungsgemäß ist aber durch Ausnutzen der Löslichkeitsunterschiede der Diamide nicht nur eine Anreicherung der Hauptfettsäurekomponenten der Ausgangsverbindungen möglich, vielmehr werden bei diesem Verfahren auch automatisch die in geringeren Mengen auftretenden Fettsäuren aufkonzentriert. Durch Verseifen der angereicherten Difettsäurediamid-Gemische und einer sich bedarfsweise anschließenden Trennung, z.B. durch Destillation, lassen sich somit Fettsäuremischungen gewinnen, die gegenüber den Ausgangsverbindungen ein deutlich verändertes Fettsäuremuster aufweisen. Unter Berücksichtigung der oben beschriebenen Gesetzmäßigkeiten für die Löslichkeiten der Difettsäurediamide ist es nach dem erfindungsgemäßen Verfahren also möglich, das Fettsäuremuster einer gegebenen Ausgangsmischung vorhersagbar zu verschieben oder aber auch Säuren, die nur in kleinen Mengen vorhanden sind, einzeln zu isolieren.

Die technische Verwertbarkeit von Ölen und Fetten wird durch die leichte Isolierbarkeit symmetrisch zusammengesetzter Diamide, wie sie durch das erfindungsgemäße Verfahren ermöglicht wird, beträchtlich erweitert. Das Verfahren ist besonders geeignet und wirtschaftlich zur Abtrennung von Fettsäuren aus Gemischen, in denen eine Fettsäure starke Dominanz aufweist und zur Auftrennung von Gemischen der industriell besonders interessanten C₁₈-Fett-säuren. Auch für die kurzkettigen Fettsäuren, insbesondere für Laurinsäure, besteht jedoch ein erheblicher industrieller Bedarf, dem nach dem erfindungsgemäßen Verfahren Rechnung getragen werden kann.

Die Erfindung ermöglicht also nicht nur die einfache und reine Darstellung bisher nur schwer zugänglicher Fettsäuren und ihrer Derivate, sondern sie erlaubt auch gleichzeitig die Bildung einer Vielzahl neuer Grundstoffe, deren technische Verwendung in der chemischen Industrie aufgrund ihrer schweren Zugänglichkeit bisher nicht in Frage kam.

Die Erfindung wird durch die nachfolgenden Beispiele näher erläutert:

### Beispiel 1

100 g Euphorbiaöl (Fettsäure-Muster: 7% Palmitinsäure, 2% Stearinsäure, 84% Ölsäure, 3% Linolsäure, 3% Linolensäure) und 9,4g 1,2-Diaminoethan wurden im Autoklaven unter Stickstoffatmosphäre 3 Stunden bei 180°C und 3 Stunden bei 100°C gerührt. Das Reaktions-produkt wurde abgetrennt und aus Methanol umkristallisiert. N,N'-Ethylenbisoleodiamid wurde mit einer Ausbeute von 71g und einer Reinheit von >90% erhalten.

### Beispiel 2

9 g Leinöl und 0,9 g 1,2-Diaminoethan wurden unter Stickstoffatmosphäre 3 Stunden bei 180°C und 3 Stunden bei 100°C im Autoklaven gerührt. Das Produkt wurde aus 75 ml Methanol umkristallisiert. Während sich in der Mutterlauge Amide mit Linol- und Linolensäure anreicherten, hatten sich im umkristallisierten Produkt Amide mit Ölsäure, Palmitinsäure und Stearinsäure angereichert.

### Beispiel 3

Umsetzung von Ricinusöl mit 1,2-Diaminoethan

5,1 g Ricinusöl und 0,5 g 1,2-Diaminoethan wurden 5 Stunden lang im Autoklaven bei 120°C unter Stickstoffatmosphäre gerührt. Das Reaktionsprodukt wurde dann aus Methanol umkristallisiert. Das auf diese Weise erhaltene N,N-Ethylenbisricinolsäurediamid besitzt eine Reinheit von >90%. (Schmelzpunkt 83 bis 85°C, Ausbeute: 2,6 g)

### Beispiel 4

Umsetzung von Ricinusöl mit 1,6-Diaminohexan

51 g Ricinusöl und 9,7 g 1,6-Diaminohexan wurden 5 Stunden lang unter Stickstoffatmosphäre bei 100°C gerührt. Das Reaktionsprodukt wurde aus 150ml Methanol umkristallisiert. Das auf diese Weise erhaltene Hexamethylenbisricinolsäurediamid besitzt eine Reinheit von >90%. (Schmelzpunkt 86 bis 88°C, Ausbeute: 32 g)

### Beispiel 5

Umsetzung von gehärtetem Ricinusöl mit 1,2-Diaminoethan

153 g gehärtetes Ricinusöl und 15 g Diaminoethan wurden 5 Stunden lang unter Stickstoffatmosphäre bei 140°C im Autoklaven gerührt. Das Reaktionsprodukt wurde dann aus Methanol umkristallisiert. Das auf diese Weise erhaltene Bis(12-hydroxystearinsäure)-N,N'-ethylendiamid besitzt eine Reinheit von >90% (Schmelzpunkt 142-145°C, Ausbeute: 106,5 g).

### Beispiel 6

Umsetzung von gehärtetem Ricinusöl mit 1,6-Diaminohexan

5,1 g gehärtetes Ricinusöl und 0,97 g 1,6-Diaminohexan wurden 5 Stunden lang unter Sickstoffatmosphäre bei 150°C im Autoklaven gerührt. Das Reaktionsprodukt wurde dann einer Lösungsmittelheißdampf-Extraktion mit Methanol unterworfen. Das auf diese Weise erhaltene Bis(12-hydroystearinsäure)-1,6-N,N'-hexamethylendiamid besitzt eine Reinheit von >90% (Schmelzpunkt 135-136°C, Ausbeute: 3,7 g)

## Patentansprüche

1. Verfahren zur Gewinnung symmetrisch zusammengesetzter Difettsäurediamide,
dadurch gekennzeichnet,
daß man Mischungen von Fettsäuren oder deren Estern, gegebenenfalls in einem geeigneten Lösungsmittel und unter Zusatz von Katalysatoren und/oder Antioxidantien zunächst mit Diaminen umsetzt, aus dem erhaltenen Reaktionsgemisch die symmetrischen Difettsäurediamide über ihre Löslichkeitsunterschiede abtrennt und falls erforderlich durch Umkristallisieren weiter aufreinigt.

2. Verfahren nach Anspruch 1, dadurch gekennzeichnet, daß als Fettsäureester Fette und Öle eingesetzt werden.

3. Verfahren nach einem der Ansprüche 1 oder 2, dadurch gekennzeichnet, daß als Fettsäureester rohe Fette und Öle eingesetzt werden.

4. Verfahren nach einem der Ansprüche 1 bis 3, dadurch gekennzeichnet, daß als Fettsäureester Fette und Öle mit einem hohen Gehalt an funktionellen Fettsäuren eingesetzt werden.

5. Verfahren nach einem der Ansprüche 1 bis 3, dadurch gekennzeichnet, daß als Fettsäureester Fette und Öle mit einem hohen Gehalt an außergewöhnlichen Fettsäuren eingesetzt werden.

6. Verfahren nach einem der Ansprüche 1 bis 5, dadurch gekennzeichnet, daß als Fettsäureester Euphorbiaöl, linol- oder ölsäurereiches Sonnenblumenöl, Ricinusöl oder hydriertes Ricinusöl, Olivenöl, Leinöl, Rapsöl, insbesondere erucasäurereiches Rapsöl, das Öl der Purgiernuß oder Seetieröle wie Fisch- oder Waltranöl eingesetzt werden.

7. Verfahren nach einem der Ansprüche 1 bis 6, dadurch gekennzeichnet, daß als Diamine primäre oder sekundäre aliphatische, cycloaliphatische, aliphatisch-aromatische oder aromatische Diamine eingesetzt werden.

8. Verfahren nach einem der Ansprüche 1 bis 7, dadurch gekennzeichnet, daß als Diamine 1,2-Diaminoethan oder 1,6-Diaminohexan eingesetzt werden.

9. Verfahren nach einem der Ansprüche 1 bis 8, dadurch gekennzeichnet, daß als Lösungsmittel für die Reaktion Toluol, Xylol oder Petrolether verwendet wird.

10. Verfahren nach einem der Ansprüche 1 bis 9, dadurch gekennzeichnet, daß die Umsetzung von Fettsäureestern und Diaminen bei Temperaturen zwischen 20 und 300°C, vorzugsweise zwischen 50 und 200°C erfolgt.

11. Verfahren nach einem der Ansprüche 1 bis 10, dadurch gekennzeichnet, daß die Umsetzung unter einer Inertgasatmosphäre erfolgt.

12. Verfahren nach einem der Ansprüche 1 bis 11, dadurch gekennzeichnet, daß als Katalysatoren Ammoniumchlorid oder p-Toluolsulfonsäure verwendet werden.

13. Verfahren nach einem der Ansprüche 1 bis 12, dadurch gekennzeichnet, daß als Antioxidantien Ascorbinsäure oder Glukose verwendet werden.

14. Verfahren nach einem der Ansprüche 1 bis 13, dadurch gekennzeichnet, daß die Abtrennung der Difettsäurediamide durch einfache oder fraktionierte Kristallisation oder durch eine Lösungsmittelheißdampf-Extraktion erfolgt.

15. Verfahren nach einem der Ansprüche 1 bis 14, dadurch gekennzeichnet, daß die Aufreinigung der Difettsäurediamide durch Umkristallisieren oder fraktioniertes Kristallisieren aus Methanol oder Ethanol erfolgt.

16. Verfahren nach einem der vorhergehenden Ansprüche, dadurch gekennzeichnet,
daß man aus den gewonnenen symmetrischen Difettsäurediamiden die jeweiligen reinen Fettsäuren durch Verseifen abspaltet.

## Claims

1. Method of producing symmetrically structured difatty acid diamides and/or the corresponding pure fatty acids,
**comprising:**
reacting mixtures of fatty acids or their esters with diamines, possibly in a suitable solvent and adding catalysts and/or antioxidants, separating the symmetric difatty acid diamides from the resulting reaction mixture by making use of their different solubilities and purifying them by recrystallisation if necessary.

2. Method as claimed in Claim 1,
characterized in that
fats and oils are used as fatty acid esters.

3. Method as claimed in any of Claims 1 or 2,
characterized in that
crude fats and oils are used as fatty acid esters.

4. Method as claimed in any of Claims 1 to 3,
characterized in that
fats and oils with a high content of functional fatty acids are used as fatty acid esters.

5. Method as claimed in any of Claims 1 to 3,
characterized in that
fats and oils with a high content of extraordinary fatty acids are used as fatty acid esters.

6. Method as claimed in any of Claims 1 to 5,
characterized in that
euphorbia oil, high-oleic and high-linoleic sunflower oil, castor oil or hydrogenated castor oil, olive oil, linseed oil, rapeseed oil, including especially rapeseed oil with high erucic acid content, the oil of Jatropha curcas or the oil of marine animals such as fish or whale oil is used as fatty acid ester.

7. Method as claimed in any of Claims 1 to 6,
characterized in that
primary or secondary aliphatic, cyclo-aliphatic, aliphatic-aromatic or aromatic diamines are used as diamines.

8. Method as claimed in any of Claims 1 to 7,
characterized in that
1,2-diaminoethane or 1,6-diaminohexane is used as diamine.

9. Method as claimed in any of Claims 1 to 8,
characterized in that
toluene, xylene or petroleum ether is used as solvent for the reaction.

10. Method as claimed in any of Claims 1 to 9,
characterized in that
the reaction of fatty acid esters and diamines takes place at temperatures between 20 and 300°C, preferably between 50 and 200°C.

11. Method as claimed in any of Claims 1 to 10,
characterized in that
the reaction takes place in an inert gas atmosphere.

12. Method as claimed in any of Claims 1 to 11,
characterized in that
ammonium chloride or toluene-p-sulphonic acid is used as catalyst.

13. Method as claimed in any of Claims 1 to 12,
characterized in that
ascorbic acid or glucose is used as antioxidant.

14. Method as claimed in any of Claims 1 to 13,
characterized in that
isolation of the difatty acid diamides is achieved by simple or fractionated crystallisation or by hot vapour extraction.

15. Method as claimed in any of Claims 1 to 14,
characterized in that
isolation of the difatty acid diamides is achieved by recrystallisation or fractionated crystallisation from methanol or ethanol.

16. Method as claimed in any of the preceding claims,
characterized in that
the respective pure fatty acids are separated from the obtained symmetric diffatty acid diamides by making use of saponification.

## Revendications

1. Procédé destiné à la préparation de diamides de diacides gras symétriques, caractérisé en ce que l'on fait d'abord réagir des mélanges d'acides gras ou leurs esters avec des diamides, le cas échéant dans un solvant approprié, en ajoutant des catalyseurs et/ou des antioxydants, en ce qu'à partir du mélange réactif obtenu l'on sépare les diamides de diacides gras symétriques en fonction de leur différence de solubilité et en ce que, si nécessaire, l'on continue à épurer par recristallisation.

2. Procédé selon la revendication 1, caractérisé en ce que l'on utilise en tant qu'esters d'acides gras des graisses et des huiles.

3. Procédé selon l'une des revendications 1 ou 2, caractérisé en ce que l'on utilise en tant qu'esters d'acides gras des graisses et des huiles brutes.

4. Procédé selon l'une quelconque des revendications 1 à 3, caractérisé en ce l'on utilise en tant qu'esters d'acides gras des graisses et des huiles à teneur élevée en acides gras fonctionnels.

5. Procédé selon l'une quelconque des revendications 1 à 3, caractérisé en ce l'on utilise en tant qu'esters d'acides gras des graisses et des huiles à teneur élevée en acides gras exceptionnels.

6. Procédé selon l'une quelconque des revendications 1 à 5, caractérisé en ce que l'on utilise en tant qu'esters d'acides gras de l'huile d'euphorbe, de l'huile de tournesol riche en acide linoléique ou oléique, de l'huile de ricin ou de l'huile de ricin hydrogénée, de l'huile d'olive, de l'huile de lin, de l'huile de colza, en particulier de l'huile de colza riche en acide érucique, de l'huile de noix de purge ou des huiles d'animaux marins telles que de l'huile de poisson ou de l'huile de baleine.

7. Procédé selon l'une quelconque des revendications 1 à 6, caractérisé en ce que l'on utilise en tant que diamines des diamines primaires ou secondaires aliphatiques, cyclo-aliphatiques, aliphatiques-aromatiques ou aromatiques.

8. Procédé selon l'une quelconque des revendications 1 à 7, caractérisé en ce que l'on utilise en tant que diamines du 1,2-diaminoéthane ou du 1,6-diaminohexane.

9. Procédé selon l'une quelconque des revendications 1 à 8, caractérisé en ce que l'on utilise en tant que solvant pour la réaction du toluène, du xylène ou de l'éther de pétrole.

10. Procédé selon l'une quelconque des revendications 1 à 9, caractérisé en ce que la réaction des esters d'acides gras et des diamines est effectuée à des températures comprises entre 20 et 300 °C, de préférence entre 50 et 200 °C.

11. Procédé selon l'une quelconque des revendications 1 à 10, caractérisé en ce que la réaction est effectuée sous une atmosphère de gaz inerte.

12. Procédé selon l'une quelconque des revendications 1 à 11, caractérisé en ce que l'on utilise en tant que catalyseurs du chlorure d'ammonium ou de l'acide p-toluique sulfonique.

13. Procédé selon l'une quelconque des revendications 1 à 12, caractérisé en ce que l'on utilise en tant qu'antioxydants de l'acide ascorbique ou du glucose.

14. Procédé selon l'une quelconque des revendications 1 à 13, caractérisé en ce que la séparation des diamides de diacides gras est effectuée par cristallisation simple ou fractionnée ou par extraction des solvants à la vapeur surchauffée.

15. Procédé selon l'une quelconque des revendications 1 à 14, caractérisé en ce que l'épuration des diamides des diacides gras est effectuée par recristallisation ou par cristallisation fractionnée à partir de méthanol ou d'éthanol.

16. Procédé selon l'une quelconque des revendications précédentes, caractérisé en ce qu'à partir des diamides de diacides gras symétriques obtenues, l'on sépare les acides gras purs respectifs par saponification.
